# EUROPEAN PATENT APPLICATION

(11) **EP 1 690 857 A1**
(43) Date of publication of application: **16.08.2006**
(21) Application number: 04819332.0
(22) Date of filing: 19.11.2004
(51) Int. Cl.: C07D 257/04, A61K 31/5377, A61K 31/41, A61K 31/4192, A61K 31/496, A61K 31/541, A61P 3/10, A61P 7/08, A61P 9/10, A61P 13/12, A61P 25/00, A61P 25/16, A61P 25/28, A61P 27/02, A61P 29/00, A61M 1/14, C07D 487/04

(54) **MODIFIED-PROTEIN FORMATION INHIBITOR**

(30) Priority: 27.11.2003 JP 2003397740
(71) Applicant: TOKAI UNIVERSITY EDUCATIONAL SYSTEM, Shibuya-ku Tokyo 1510063 (JP); Miyata, Toshio, Isehara-shi, Kanagawa 259-1132 (JP); Kurokawa, Kiyoshi, Shinjuku-ku, Tokyo 162-0061 (JP)
(72) Inventor: MIYATA, Toshio, Isehara-shi, Kanagawa 259-1132 (JP); KUROKAWA, Kiyoshi, Shinjyuku-ku, Tokyo 162-0061 (JP)
(74) Representative: HOFFMANN EITLE
(86) International application number: PCT/JP2004/017267
(87) International publication number: WO 2005/051930

(57) **Abstract**

[PROBLEMS] To provide a inhibitor of protein modification products formation that exibits intense and excellent protein modification products formation inhibiting effects without causing any blood pressure drop.

[MEANS FOR SOLVING PROBLEMS] There is provided a inhibitor of protein modification products formation comprising as an active ingredient a compound consisting of a tetrazole ring having, via methylene, various substituents, especially compound (I) or (II) of the following formula: 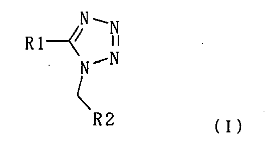 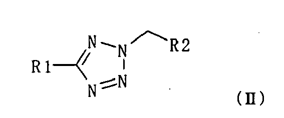 (wherein R1 and R2 represent monovalent organic groups identical with or different from each other). This inhibitor of protein modification products formation is useful in the prevention and treatment of diseases associated with AGEs and ALEs, for example, used as a renal tissue protectant alone or in mixture in a peritoneal dialyzing solution or hemodialysate.

## Description

### TECHNICAL FIELD OF THE INVENTION

The present invention relates to a inhibitor of protein modification products formation, particularly to a medicament inhibiting the formation of protein modification products such as advanced glycation end products (AGEs) and advanced lipoxidation end products (ALEs), which are formed by the reaction with carbonyl compounds under non-enzymatic conditions.

### BACKGROUND OF THE INVENTION

Glycation represents the chain reactions starting from the non-enzymatic reaction between the amino moiety on peptides or proteins and the carbonyl moiety on reducing sugars (Maillard reaction; cf. Reference 1) and are divided roughly into the initial stage and the later stage. The initial stage comprises a reversible reaction depending on the concentration of sugars and the reaction time wherein the amino moiety and the carbonyl moiety are non-enzymatically reacted to form Schiff bases, followed by Amadori rearrangement to form Amadori compounds.

In the later stage, the Amadori compounds formed in the initial stage are irreversibly subjected to dehydration, condensation, cyclization, oxidation, fragmentation, polymerization, rearrangement, etc. to give finally protein modification products called "AGEs". By auto-oxidation of sugars and the like, there are produced highly reactive dicarbonyl compounds such as 3-deoxiglucosone (hereinafter referred to as "3-DG"), glyoxal (hereinafter referred to as "GO") and methylglyoxal (hereinafter referred to as "MGO"), which may be further reacted with proteins to form AGEs modified at the lysine or arginine residues of the proteins in many cases.

Under the oxidation stress conditions, sugars, lipids, amino acids, etc. present abundantly in living bodies are oxidized to highly reactive carbonyl compounds. The produced GO, MGO, arabinose, glycol aldehyde, etc. are served as precursors of AGEs. Dehydroascorbic acid, which is formed by oxidation of ascorbic acid, is also served as a precursor of AGEs. These precursors have a carbonyl group, which is reacted non-enzymatically reacted with the amino moiety on proteins to give Schiff's bases and then form AGEs (cf. Reference 2).

Under the oxidation stress condition, lipoperoxidation also proceeds to form various carbonyl compounds such as malondialdehyde, hydroxynonenal and acrolein (cf. Reference 3). These carbonyl compounds react with the amino moiety or the like on proteins to form protein modification products called ALEs such as malondialdehyde-modified lysine and modified hydroxynonenal (cf. Reference 2).

In addition, amino acids such as serine and threonine are oxidized to form carbonyl compounds such as acrolein and GO, followed by conversion into protein modification products (cf. Reference 4). A large number of carbonyl compounds are formed by the oxidative pathway, but some carbonyl compounds such as 3-DG are formed through the non-oxidative pathway.

As the known pathways for formation of AGEs, there are (i) the pathway of conversion of Shiff's bases or Amadori compounds into AGEs through 3-DG, (ii) the pathway of oxidative conversion of Shiff's bases into glycolaldehyde alkylimines, followed by conversion of the latter into AGEs via aldoamines, (iii) the pathway conversion of aldoamines into AGEs via glyoxal monoalkylimines, (iv) the pathway of conversion of amarori comounds into MGO through 2,3-enediol, followed by conversion of said MGO into AGEs, and (v) others.

It has recently been revealed that carboxymethyl-lysine as one of the AGEs is produced from GO, which is formed by lipoxidation of unsaturated fatty acids. It is thus considered that the glycation/oxidation reaction and the lipoxidation reaction occur on the common basis.

As understood from the above, carbonyl compounds produced through the oxidative or non-oxidative pathway from sugars, lipids, amino acids and ascorbic acid, modify proteins non-enzymatically and finally give protein modification products such as AGEs and ALEs. In particular, the increased state of the protein modification reaction by carbonyl compounds formed via a plurality of reaction pathways is called the protein modification due to excessive carbonyl, i.e. "carbonyl stress".

Known AGEs include pentosidine (cf. Reference 5), crossrine (cf. Reference 6), X1 (fluorolink), pyropyridine (cf. Reference 7), pyrarine (cf. Reference 8), carboxymethyl-lysine (cf. Reference 9), imidazolone compounds (cf. Reference 10), carboxyethyl-lysine (cf. Reference 11), MGO dimer (cf. Reference 12), GO dimer (cf. Reference 13), imidazolysine (cf. Reference 14), argupyrimidine (cf. Reference 15), etc.

AGEs receptors as heretofore cloned include RAGE (cf. Reference 16), macrophage scavenger receptor class A (cf. Reference 17), galectin 3 (cf. Reference 18), OST-48 and 80K-H (cf. Reference17), etc.

It is reported that in the blood vessel tissue, RAGE (a cellular membrane penetration type protein belonging to the immunoglobulin superfamily) is bonded to AGE, thereby active oxygen is generated in the cell to activate the p21ras/MAPK pathway (cf. Reference 19) so that the activation of the transcription factor NF-κβ is induced to lead the expression of angiopathy associated factors such as VCAM-1 (cf. Reference 20). It is also reported that AGEs control the proliferation of endothelial cells in finer vessels via RAGE, control the proliferation of pericytes playing an important roll in homeostasis and produce a toxic effect (cf. Reference 21).

In addition, it is reported that AGEs act directly onto endothelial cells in finer vessels via RAGE to promote neoangiogenesis and inhibit the production of PG12 for thrombus tendency (cf. Reference 22). For further interests, enhancement of the substrate production in mesanginal cells, enhancement of the monocyte migration ability, release of inflammatory cytokines from macrophages, acceleration of the collagenase production in synovial cells, activation of osteoclasts, proliferation of vascular smooth muscle cells, acceleration of platelet aggregation, NO activity and its suppression of the smooth muscle relaxation are reported as the physiological activities of AGEs and ALEs (cf. Reference 23).

Diseases associated with AGEs include (i) nephropathy as a complication of diabetes (cf. Reference 24), nervous disorder (cf. Reference 25), retinopathy (cf. Reference 21) and cataract, (ii) arteriosclerosis (cf. Reference 26), (iii) dialysis amyloidosis as a complication of dialysis (cf. Reference 27) and peritoneal sclerosis in peritoneal dialysis patients, (iv) Alzheimer's disease as a central neurological disease (cf. Reference 28), Pick's disease and Parkinson disease, (v) rheumatoid arthritis (cf. Reference 29), (vi) sunlight elastic fibrosis, (vii) aging, (viii) renal failure (cf. Reference 30), etc. In addition, it is reported that in case of diabetes, AGEs prevent the vasodilation derived from blood vessel endothelial cells (cf. Reference 31), and promote renal sclerosis (cf. Reference 32).

From the above, it is understood that protein modification products such as AGEs afford an adverse effect on living bodies directly or via receptors.

On the other hand, it is known that the blood concentration of AGEs are increased with the reduction of the renal function. The reduction of the renal function results in the accumulation of carbonyl compounds considered as having a molecular weight of not more than 5kDa. In case of pentosidine or pyrarine, those can be present in a free form, but a large portion of them present in a binding form to serum albumin or the like (cf. Reference 33). In addition, it is reported that the blood level of pentosidine is strongly affected by the filtration function of glomeruli (cf. Reference 34).

In this way, a large portion of AGEs is eliminated from kidney, and their blood concentration is kept lower during good health. However, when the renal function is reduced, they act as a uremic toxin to produce chronic bioactivities.

Dialysis therapy can remove AGEs in a free form but hardly remove those in a binding form to proteins or in an intramolecular bridging form (cf. Reference 35). Therefore, the accumulation of modified forms in living bodies is increased with the progression of renal failure. Further, in addition to the fundamental process where sugars are reacted in living bodies, AGEs in a free form supplied by diets as well as highly active intermediates such as 3-DG, GO and MGO formed from Amadori compounds and the like previously produced in living bodies react with proteins in succession to enhance the production of AGEs. Furthermore, the contact of blood to a dialysis membrane results, for instance, in activation of complements and leucocytes to enhance the generation of free radicals. Thus, dialysis therapy itself enhances oxidation and makes one of the causes for production of AGEs generation.

Accordingly, it is important in dialysis therapy to remove free form substances at an early stage of dialysis and suppress the generation of AGEs in a binding form as much as possible. Since it is difficult to remove AGEs in a binding form by dialysis therapy as stated above, development of a medicament which suppresses formation of protein modification products is highly desired for dialysis therapy.

Further, it is believed that not only reduction of renal function but also fall of anti-oxidation protective mechanism associated with renal failure is concerned with accumulation of protein modification products. In patients with renal failure, unbalance of such anti-oxidation abilities is suggested (cf. Reference 40) as the increase of oxidized glutathione against reducing glutathione in blood (cf. Reference 36), the reduction of activity of glutathione dependent enzymes, the decrease of preservation term renal failure plasma glutathione peroxidase (cf. Reference 37), the decrease of blood glutathione (cf. Reference 38) and the increase of activity of plasma superoxide dismutase against the decrease of selenium concentration in plasma (cf. Reference 39).

Furthermore, it is reported that in patients with chronic renal failure, remarkable amounts of highly reactive carbonyl compounds and AGEs are generally accumulated in blood and tissues regardless of hyperglycemia (cf. Reference 41). In renal failure, carbonyl compounds are placed under a state of high load (carbonyl stress) by non-enzymatic chemical reaction so that protein modification products are increased. This is considered to have been caused by modification of proteins with carbonyl compounds produced from sugars and lipids (cf. Reference 42).

Accordingly, suppression of the production of protein modification products caused by various factors may accomplish alleviation of tissue injury and prevent or treat the conditions associated with protein modification products such as AGEs.

Dialysis for patients with chronic renal failure includes hemodialysis and peritoneal dialysis. In case of peritoneal dialysis, debris in blood is excreted into peritoneal dialysate through a peritoneal membrane. Peritoneal dialysate of high osmotic pressure, which contains glucose, icodextrin, amino acid or the like, is effective in collecting such highly reactive carbonyl compounds accumulated in blood of patients with renal failure as carbonyl compounds derived from carbohydrates (e.g. arabinose, GO, MGO, 3-DG), carbonyl compounds derived from ascorbic acid (e.g. dehydroascorbic acid) and carbonyl compounds derived from lipids (e.g. hydroxynonenal, malondialdehyde, acrolein) through a peritoneal membrane therein.

Further, it is known that highly reactive carbonyl compounds (e.g. 3-DG, 5-hydroxymethylfurfural, formaldehyde, acetaldehyde, GO, MGO, levulinic acid, furfural, arabinose) are formed in a peritoneal dialysate during the sterilization or storage of the peritoneal dialysate (cf. Reference 43).

Therefore, the concentration of carbonyl compounds in the peritoneal dialysate increases, and the formation of protein modification products enhances. As the result, the function of the peritoneal membrane is reduced, and thereby reduction of the water removing ability and progression to peritoneal sclerosis would be caused (cf. Reference 44).

In fact, it is demonstrated by the immunohistological study of endothelium and mesothelium that in patients with peritoneal dialysis, introduced glucose makes a carbonyl stress condition in the peritoneal cavity (cf. Reference 45).

In this way, it is presumed that in patients with dialysis, formation of protein modification products by carbonyl compounds causes the morphological alteration of peritoneum and the reduction of the function (i.e. water removal function) resulting therefrom.

Taking into consideration the above facts and various morbid conditions such as renal failure in combination, it is believed that the accumulation of carbonyl compounds is one of the causes for enhancement of the AGEs formation (cf. Reference 46). Thus, suppression of the AGEs formation is considered as an effective measure for treatment of the conditions associated with AGEs.

A typical example of AGEs formation inhibitors is aminoguanidine, which is considered to inhibit AGEs formation by reaction with dicarbonyl compounds such as 3-DG generated from glucose, Shiff's bases or Amadori compounds to form thiazolines. Analysis using diabetes animal models confirmed that said compound is effective in delaying the progression of diabetic nephropathy (cf. Reference 47), retinopathy (cf. Reference 48) and cataract (cf. Reference 49).

Other examples are pyridoxamine derivatives (e.g. pyridorine). In case of OPB-9195 (i.e. (±)2-isopropylidene-hydrazon-4-oxo-thiazolydin-5-yl-acetanilide), the nitrogen atom in the hydrazine moiety is reacted with a carbonyl group to form a stable structure. Thus, it captures a reactive carbonyl group in a free form or a binding form to protein (cf. Reference 50) and therefore can prevent the formation of not only AGEs but also ALEs in vitro. Since biguanide compounds such as metformin or buformin can also capture carbonyl compounds (cf. Reference 51), they have a possibility of being used as AGEs forming inhibitors. Further, the use of AGEs inhibitors capable of cleaving the bridge as a characteristic of AGEs and the enzymes capable of degrading Amadori compounds (i.e. Amadoriase) are proposed.

Study is also made on the possibility of prevention of the AGEs and/or ALEs formation by removal of carbonyl compounds. For removal of carbonyl compounds, there are available several enzymes and enzymatic pathways, of which examples are aldol reducing enzymes and aldehyde dehydrogenase and glyoxalase pathway (cf. Reference 52). Redox co-enzymes such as reducing glutathione (GSH) and NAD(P)H. are important factors for activation of those pathways.

Lowering of these removing pathways leads to increasing of numerous carbonyl compounds at the same time. Carbonyl compounds such as MGO and GO react with the thiol group of GSH and, as the result, they are metabolized with an enzyme, i.e. glyoxalase. NAD(P)H activates the glutathione reducing enzyme and enhances the GSH level. Namely, it is believed that the removal system of carbonyl compounds is inhibited by lowering of GSH or NAD(P)H due to unbalance of the intracellular redox mechanism, which leads to accumulation of AGEs and ALEs. In case of diabetes, it is suggested that the polyol pathway is activated by hyperglycemia, NAD(P)H and GSH are reduced and the removal system of carbonyl compounds is lowered.

If reduction in the concentration of thiols such as GSH and NAD(P)H lowers the removal of carbonyl compounds, and thereby causes the formation of AGEs or ALEs as stated above, there is a possibility that carbonyl compounds would be decreased by increasing the thiol level. For this purpose, the supplementation of thiol groups with GSH, cysteine, acetylcysteine, etc., the lowering of the GSH demand with vitamin E, ubiquinol, etc. and the inhibition of the polyol system with aldose reducing enzyme inhibitors are proposed. Trapping of carbonyl compounds by the use of aminoguanidine, pyridoxamine, hydrazine, biguanide compounds or SH-containing compounds is also proposed (cf. Patent Reference 1).

As stated above in detail, the inhibitions of formation of AGEs and ALEs are the way for prevention and treamtent of diseases associated with them.
Patent Reference 1: WO 00/10606
Reference1: Maillard, L. C.et al., "Compt. Rend. Soc. Biol.", (FR), 1912, Vol.72, p599
Reference 2: Miyata, T.et al., "Kidney Int.", (US), 1999, Vol.55, p389-399
Reference 3: Esterbauer, H.et al., "Free Radic. Biol. Med.", (US), 1991, Vol.11, p81-128
Reference4: Anderson, M. M.et al., "J. Clin. Invest.", (US), 1997, Vol.99, p424-432
Reference 5: Sell, D. R.et al., "J. Biol. Chem.", (US), 1989, Vol.264, p21597-21602
Reference 6: Nakamura, K.et al., "J. Chem. Soc. Chem. Commun.", (GB), 1992, Vol.15, p992-994
Reference 7: Hayase, F.et al., "Biosci. Biotech. Biochem " (JP), 1994, Vol.58, p1936-1937
Reference 8: Njoroge, F. G.et al., "Carbohyd. Res.", (NL), 1987, Vol.167, p211-220
Reference 9: Ahmed, M. U.et al., "J. Biol. Chem.", (US), 1986, Vol.261, p4889-4894
Reference 10: Hayase, F.et al., "Biosci. Biotech. Biochem.", (JP), 1995, Vol.59, p1407-1411
Reference 11:Ahmed, M. U.et al., "Biochem. J.",(GB), 1997, Vol.324, p565-570
Reference 12: Brinkmann, E.et al., "J. Chem. Soc. Perkin. Trans.", (GB), 1995, Vol.2, p1-2
Reference 13: Well-Knecht, K. J.et al., "J. Org. Chem.", (US)1995, Vol.60, p6246-6247
Reference 14: Nagaraj, R. H.et al., "J. Biol. Chem.", (US), 1996, Vol.271, p19338-19345
Reference 15: Shipanova, I. N.et al., "Arch. Biochem. Biophys.",(US), 1997, Vol.334, p29-36
Reference 16: Neeper, M.et al., "J. Biol. Chem.", (US), 1992, Vol.267, p14998-15004
Reference 17: Suzuki, H.)et al.,"Nature",(GB)1997, Vol.386, p292-295
Reference 18: Vlassara, H et al., "Molecular Medicine",(US), 1995, Vol.1 , p634-646
Reference 19: Lander, H. M.et al., "J. Biol. Chem.", (US), 1997, Vol.272, p17810-17814
Reference 20: Chappey, O.et al., "Eur. J. Clin. Invest.",(GB), 1997, Vol.27, p97-108
Reference 21: Yamagishi, S.et al., "Biochem. Biophys. Res. Commun., (US), 1995, Vol.213, p681-687
Reference 22: Yamagishi, S.et al., "FEBS Lett.",(NL), 1996, Vol.384, p103-106
Reference 23: Doi, T.et al., "Proc. Natl. Acad. Sci. USA)", (US), 1992, Vol.89, p2873-2877
Reference 24: Horie, K.et al., "J. Clin. Invest.",(US), 1997, Vol.100, p2995-3004
Reference 25: Sugimoto, K.et al., "Diabetologia", (DE), 1997, Vol.40, p1380-1387
Reference 26: Park, L.et al., "Nat. Med.",(US), 1998, Vol.4, p1025-1031
Reference 27: Miyata, T.et al., "J. Clin. Invest.",(US)1993, Vol.92, p1243-1252
Reference 28: Smith, M. A.et al., "Proc. Natl. Acad. Sci. USA", (US), 1994, Vol.91(12), p5710-5714
Reference 29: Miyata, T.et al., "Biochem. Biophys. Res. Commun.", (US), 1999, Vol.244, p45-49
Reference 30: Makita, Z.et al., "N. Engl. J. Med.", (US), 1991, Vol.325, p836-842
Reference 31: Bucala, R.et al., "J. Clin. Invest.", (US), 1991, Vol.87, p432-438
Reference 32: Vlassara, H.et al., "Proc. Natl. Acad. Sci. USA", (US), 1994, Vol.91, p11704-11708
Reference 33: Miyata, T.et al., "J. Am. Soc. Nephrol.", (US), 1996, Vol.7, p1198-1206
Reference 34: Sugiyama, S.et al., "J. Am. Soc. Nephrol.", (US), 1998, Vol.9, p1681-1688
Reference 35: Miyata, T.et al., "Kidney Int.", (US), 1996, Vol.49, p1304-1313
Reference 36: Canestrari, F.et al., "Acta Haematol.", (CH), 1994, Vol.91, p187-193
Reference 37: Ueda, Y.et al., "Biochem. Biophys. Res. Commun.", (US), 1998, Vol.245, p785-790
Reference 38: Canestrari, F.et al., "Acta Haematol.", (CH), 1994, Vol.91, p187-193
Reference 39: Richard, M. J.et al., "Nephron", (CH), 1991, Vol.57, p10-15
Reference 40: Jadoul, M.et al., "Kidney Int.", (US), 1999, Vol.55, p2487-2492
Reference 41: Miyata, T.et al., "Kidney Int.", (US)1997, Vol.51, p1170-1181
Reference 42: Miyata, T.et al., "Kidney Int.", (US), 1999, Vol.55, p389-399
Reference 43: Richard, J. U.et al., "Fund. Appl. Toxic.", (US), 1984, Vol.4, p843-853
Reference 44: Miyata, T.et al., "Kidney Int.", (US), 2000, Vol.58, p425-435
Reference 45: Yamada, K.et al., "Clin. Nephrol.", (DE), 1994, Vol.42, p354-361
Reference 46: Miyata, T.et al., "Nephrol. Dial. Transplant.", (GB), 1997, Vo1.12, p255-258
Reference 47: Edelstein, D.et al., "Diabetologia, (DE), 1992, Vo1.35, p96-101
Reference 48: Hammes, H. P.et al., "Proc. Natl. Acad. Sci. USA", (US), 1991, Vol.88, p11555-11561
Reference 49: Matsumoto, K.et al., "Biochem. Biophys. Res. Commun.", (US), 1997 Vol.241, p352-354
Reference 50: Nakamura, S.et al., "Diabetes", (US), 1997, Vol.46, p895-899
Reference 51: Beisswenger, P. J.et al., "Diabetes, (US), 1999, Vol.48, p198-202
Reference 52: Thornalley, P. J. et al., "Endocrinol. Metab.", (US), 1996, Vol.3, p149-166

### SUMMARY OF THE INVENTION

### Problems

It is known that aminoguanidine and OPB-9195 known as inhibitor of protein modification products formations cause serious vitamin B6 deficiency when they are administrated in organisms. In the course of an extensive study to overcome such defect, the present inventors found that said vitamin B6 deficiency is attributed to the trapping of vitamin B6 molecules in blood by said inhibitor of protein modification products formations.

Based on the above finding, a further study was conducted to provide a medicament for preventing and treating a disease associated with a protein modification products(s) (i.e., AGEs and/or ALEs) produced by reacting with a carbonyl compound under the non-enzymatic condition, and suppressing the vitamin B6 deficiency as a side effect. As the result, the present inventors found that compounds having a tetrazole moiety, particularly angiotensin II receptor blockers having a tetrazole moiety and their pharmaceutically acceptable salts inhibit effectively the formation of protein modification products such as AGEs, ALEs, etc. On the basis of this finding, an invention on inhibitor of protein modification products formations comprising said compounds as the active ingredient was completed (Japanese Patent Application No. 2001-147115).

Angiotensin II receptor blockers exhibit an excellent effect as inhibitor of protein modification products formations. However, due to their blood pressure lowering activity, their use is restricted in patients with a disease in which the change of blood pressure is undesirable. One of the aims of the present invention is to provide a inhibitor of protein modification products formation without lowering of blood pressure.

### Means for solving problems

As a result of the extensive study aiming at providing a inhibitor of protein modification products formation having a better effect on the basis of the knowledge and information as stated above, it has been found that compounds having various substituents on a tetrazole ring with intervention of a methylene group exhibit potent and excellent protein modification products formation inhibition and are useful as inhibitor of protein modification products formations without blood pressure depression.

Namely, the present invention provides a inhibitor of protein modification products formation comprising as an active ingredient a compound having a protein modification products formation inhibiting effect with suppress of vitamin B6 deficiency as an adverse effect, preferably further not accompanied with depression of blood pressure. The scope of this invention covers specifically following technical embodiments:spects:
(i) a inhibitor of protein modification products formation comprising as an active ingredient a 5-substituted tetrazole ring compound in a free or salt form, the tetrazole ring having a methylene-containing group at the 1- or 3-position.
(ii) a inhibitor of protein modification products formation according to (i), wherein the compound as the active ingredient is selected from compounds of formula (I): 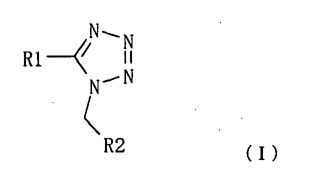 [wherein R1 and R2 are the same or different monovalent organic group]
   in a free or salt form;
(iii) a inhibitor of protein modification products formation according to (i), wherein the compound as the active ingredient is selected from compounds of formula (II): 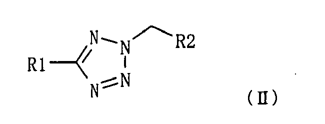 [wherein R1 and R2 are the same or different monovalent organic group]
   in a free form or salt form;
(iv) a inhibitor of protein modification products formation according to (ii) or (iii), wherein R1 is an optionally substituted phenyl group and R2 is an optionally substituted heterocyclic group having not more than 10 ring atoms;
(v) a inhibitor of protein modification products formation according to (ii) or (iii), wherein R1 is an optionally substituted phenyl group and R2 is a lower alkyl group, a lower alkoxy group or a hydroxy group;
(vi) a inhibitor of protein modification products formation according to (ii) or (iii), wherein R1 is a substituted or unsubstituted phenyl group and R2 is a morpholino group;
(vii) a inhibitor of protein modification products formation according to (ii) or (iii), wherein R1 is a substituted or unsubstituted phenyl group and R2 is a 5-methyl-3a,6a-dihydro-1H-pyrrolo[1,2,3]-triazole-4,6-dione group;
(viii) a inhibitor of protein modification products formation according to (ii) or (iii), wherein R1 is a substituted or unsubstituted phenyl group and R2 is a hydroxymethyl group;
(ix) a inhibitor of protein modification products formation according to any one of (i) to (viii), wherein the protein modification products is selected from AGEs and ALEs and combination thereof;
(x) a inhibitor of protein modification products formation according to (ix), wherein the protein modification products is AGE;
(xi) a inhibitor of protein modification products formation according to (x), wherein AGE is pentosidine;
(xii) a renal tissue protecting agent comprising the inhibitor of protein modification products formation according to any one of (i) to (viii);
(xiii) a peritoneal dialysate comprising the inhibitor of protein modification products formation according to any one of (i) to (viii);
(xiv) a hemodialysis fluid comprising the inhibitor of protein modification products formation according to any one of (i) to (viii);
(xv) a method for reduction of the amount of a carbonyl compound(s) in a liquid sample, which comprises contacting the inhibitor of protein modification products formation according to any one of (i) to (viii) with the liquid sample;
(xvi) a method for suppression of the formation of a protein modification products in the blood or peritoneal dialysate of a patient, which comprises contacting the inhibitor of protein modification products formation according to any one of (i) to (viii) with said blood or peritoneal dialysate;
(xvii) a method for treatment of a disease mediated by the formation of a protein modification products, which comprises administering a therapeutically effective amount of the inhibitor of protein modification products formation according to any one of (i) to (viii) to a patient in need of such treatment;
(xviii) use of the inhibitor of protein modification products formation according to any one of (i) to (viii) for preparation of a medicament for treatment of a disease mediated by the formation of a protein modification products.

### DETAILED DESCRIPTION OF THE PREFERRED EMBODIMENTS

The present invention provides a inhibitor of protein modification products formation comprising as an active ingredient a 5-substituted tetrazole ring compound in a free or salt form, wherein the tetrazole ring has a methylene-containing group at the 1 or 3-position. Specifically, the active ingredient may be selected from compounds of formula (I): 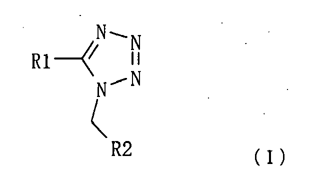 [wherein R1 and R2 are the same or different monovalent organic group]
in a free or salt form and compounds of formula (II): 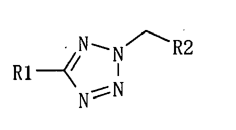 [wherein R1 and R2 are the same or different monovalent organic group]
in a free or salt form. The active ingredient has a protein modification products formation inhibiting effect with suppression of vitamin B6 deficiency as an adverse effect and further with less variation of blood pressure and is suitable for the use as a medicament.

The term "protein modification products" herein used is intended to mean a protein modification products (e.g., AGEs, ALEs, etc.) produced by the reaction with a carbonyl compound under a non-enzymatic condition formed by the reaction with carbonyl compounds under the non-enzymatic condition and cover AGEs and ALEs unless otherwise stated specifically. The protein modification products may be thus AGEs or ALEs, or their combination. Examples of AGEs are pentosidine, crossrine, X1 (fluorolink), pyropyridine, pyrarine, carboxymethyl-lysine, imidazolone compounds, carboxyethyl-lysine, MGO dimer, GO dimer, imidazolidine and argupyrimidine. Examples of ALEs are malondialdehydolysine, modified hydroxynonenal, etc.

The term "carbonyl compound" is intended to mean a compound having a carbonyl group causing protein modification regardless of being derived from organisms or non-organisms and covers dicarbonyl compounds. Examples of the carbonyl compound include arabinose, GO, MGO, 3-DG, glycolaldehyde, dehydroascorbic acid, hydroxynonenal, marondialdehyde, acrolein, 5-hydroxymethylfurfural, formaldehyde, levulinic acid, furfural, etc.

The term "vitamin B6 deficiency" refers to several disease caused by the deficiency of vitamin B6, and includes angular cheilitis, mouth inflammation, glossitis, chelitis, acute and chronic eczema, contact dermatitis, peripheral neuritis, nerve disorder, anemia and hypolymphemia.

A compound having 5-substituted tetrazole ring in free or salt form, wherein said tetrazole ring has methylene included group on 1 or 3 position as active ingredient of "inhibitor of protein modification products formation" finally suppress the formation of the protein modification products regardless of in vivo , ex vivo and/or in vitro. The term "finally suppress" may caused by their effect to trap the carbonyl compounds or by suppressing the reaction to form protein modification products. It allows any mechanism to suppress finally the formation of protein modification products, there is no limitation of their mechanism. In addition, the term "inhibitor" or "protectant" includes medicament for preventive and/or therapeutic use.

5-substitutede tetrazole ring compounds used as active ingredient of inhibitor of protein modification products formation according to this invention are represented by formula (I) or (II) as described above.

In formula (I) and (II), R1 represents unsubstituted or substituted aromatic ring (includes heterocyclic ring) group. "aromatic ring group" involves not more than 20 ring constituent atom (hetero atoms such as oxygen, sulfate or nitrogen may be present therein and the number of them is not more than 3), in particular are preferred aryl comprising 6 to 10 ring constituent carbon atoms (for example phenyl or naphthyl).

The substituents may be selected from one or more of for example, lower alkyl (such as methyl, ethyl, propyl), lower alkenyl (such as binyl, allyl), lower alkoxy (such as methoxy, ethoxy, propoxy), lower alkenyloxy (such as binyloxy, allyloxy), lower alkanoyl (such as acetyl, propyonyl, buthyryl), halo (lower) alkyl (such as monochloro methyl, dichloro methyl, trifluoromethyl, dichloroethyl), carboxyl, (lower) alkoxycarbonyl (such as methoxycarbonyl, ethoxycarbonyl, propoxycarbonyl), carboxy (lower) alkyl (such as carboxymethyl, carboxyethyl, carboxypropyl), halogen (such as chlorine, bromide, iodine, fluorine), nitro, amino, hydroxy, hydroxysulfonyl, aminosulfonyl, oxadiazolyl, thiadiazolyl. The number of substituents is not limited, however, usually not more than 3.

R2 represents monovalent organic group. The "monovalent organic group" includes non-substituted or substituted hydrocarbon, hydroxy, thiol, carboxyl, lower alkoxycarbonyl, carboxy alkyl, amino, lower alkanoylamino, aryloxyamino, 3 to 7 membered heterocyclic group. The "hydrocarbon group" includes chained or cyclic, aliphatic, cycloaliphatic or aromatic hydrocarbon group comprising not more than 30 carbon atoms, and is exemplified alkyl, alkenyl, alkynyl, cycloalkyl, cycloalkenyl, aryl group. The "3 to 7 membered heterocyclic group " includes 3 hetero atoms as ring constituent atoms, and includes for example pyrrolidino, piperidino, morpholino, thiamorpholino. The kind and number of substituent are as defined in R1. The organic group preferred as R2 is optionally further substituted with lower alkyl, oxy or etc. hydroxy, morpholino and pyrrolotriazolyl.

In addition, the term "lower" in the context of words alkyl, alkoxy and alkanoyl as stated above usually refers to the group comprising not more than 8, preferably not more than 5 carbon atoms.

The compound (I) of this invention includes specifically following:

| Table 1 | | |
|---|---|---|
| Number | Constitutional formula | Chemical name |
| 1 | 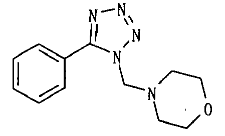 | 4-(5-phenyl-tetrazol-1-yl methyl)-morpholine |
| 2 | 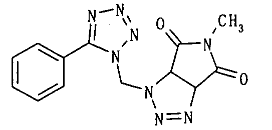 | 5-methyl-1-(5-phenyl-tetrazol-1-yl methyl)3a,6a-dihydro-1H-pyrrolo[3,4-d][1,2,3]triazol-4,6-dion |
| 3 | 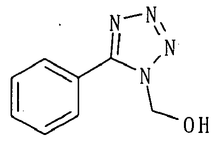 | (5-phenyl-tetrazol-1-yl)methanol |
| 4 | 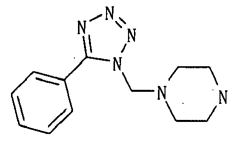 | 1-(5-phenyl-tetrazol-1-yl methyl)-piperazine |
| 5 | 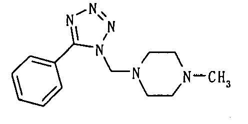 | 1-methyl-4-(5-phenyl-tetrazol-1-yl methyl)-piperazine |
| 6 | 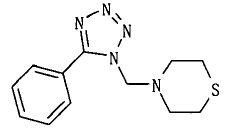 | 4-(5-phenyl-tetrazol-1-yl methyl)-thio morpholine |
| 7 | 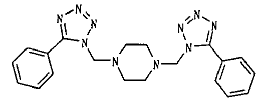 | 1,4-bis-(5-phenyl-tetrazol-1-yl methyl)-piperazine |

To prepare the compound (I) or (II) of this invention, generally, depending on 5-substituted tetrazole and the kind of methylene included group which should introduce on 1 or 3 position, do suitable chemical reaction known per se.

For example, morpholine is reacted with 5-phenyl tetrazole in presence of formalin to prepare 4-(5-phenyltetrazol-1-yl methyl)-morpholine. The reaction is usually done by treating the both in organic solvate (methanol) at 5 °C.

The compound (I) or (II) of this invention itself shows a protein modification products formation inhibiting effect without vitamin B6 deficiency as an adverse effect in organisms. This fact can be confirmed by the following test:
(A) Test to prove that the compound (I) or (II) itself exhibits a protein modification products formation inhibiting effect:
   To a plasma sample taken from a dialysis patient without diabetes, the compound of this invention is added, and after a certain period of time, an amount of pentosidine formed is determined using pentosidine, a typical example of AGEs, as an index.
(B) Test to prove that the compound (I) or (II) does not cause vitamin B6 deficiency:
   To a solution of vitamin B6, the compound of this invention is added, and after a certain period of time, an amount of vitamin B6 remained is determined.

The inhibitor of protein modification products formation of this invention comprising the compound (I) or (II) as an active ingredient is usable to prevent and/or treat for following conditions: renal failure, diabetic complication (nephropathy, nerve disorder, retinopathy, cataract, etc.), arteriosclerosis, dialysis amyloidosis which is complication of dialysis, and peritoneal screlosis in peritoneal dialysis patient, Alzheimer's disease which is central neurological disease, Pick's disease and Parkinson disease, rheumatoid arthritis, sunlight erastic fibrosis, aging and etc. Said inhibitor is particularly usable to prevent and/or treat renal disorder.

The compound (I) of this invention may directly or with treatment such as dilution with water, use when it is used as preventive agent or therapeutic agent, and may use in combination with medicinal drugs or quasi drugs. The blending quantity in this case is selected suitable depending on the condition or the product and 0.001 to 50 % by weight, especially 0.01 to 10 % by weight of the compound is usually suitable when it is administered systemically. Since sufficient preventive or therapeutic effect may not achieve when it comprises less than 0.001% by weight, or since property of the product such as stability or flavor may be impaired when it comprises more than 5% by weight, they are not preferred.

The compound (I) of this invention may be comprised in free or salt form. The salt includes pharmaceutically acceptable salt, for example salt with inorganic or organic base, acid addition salt such as inorganic acid, organic acid, and basic or acidic amino acid addition salt. The salt with inorganic base includes for example alkali metal (such as sodium or potassium) salt, alkali earth metal (such as calcium, magnesium) salt, alminum salt and ammonium salt. The salt with organic base includes for example salts with primary amine (such as ethanol amine), secondary amine (such as diethyl amine, diethanol amine, dicyclohexyl amine, N,N' -dibenzylethylen diamine),and tertiary amine (such as trimethylamine, triethylamine, biridine, picoline, triethanol amine).

The salt with inorganic acid is exemplified salts with hydrochloric acid, hydrobromic acid, nitric acid, sulfuric acid and phosphoric acid, and the salt with organic acid is exemplified salts with formic acid, acetic acid, lactic acid, trifluoro acetic acid, fumaric acid, oxalic acid, tartaric acid, maleic acid, benzoic acid, citric acid, succinic acid, malic acid, methanesulfonic acid, ethanesulfonic acid, benzenesulfonic acid and p-toluenesulfonic acid. In addition, the salt with basic amino acid is exemplified salts with arginine, lysine and ornithine, and the acidic amino acid is exemplified salts with aspargine acid and glutamic acid.

The compound (I) or (II) of this invention is optionally used in combination with known agent such as amino guanidine, pyridoxamine derivative, OPB-9195, biguanide compound, bridge formation inhibitor, enzyme degrading Amadori compounds, GSH, cystein, acetyl cystein, vitamin E, ubiquinol, aldose reduction enzyme inhibitor, carbonyl compounds trapping agent to enhance sustentation of protein modification products formation inhibiting effect. In addition, identify material(s) which deactivate or degrade the compound (I) or (II), select materials which inhibit the identified materials and selected material are used together of blended to obtain the stability of active ingredient in the composition.

The administration route of the medicament of this invention may be selected from transmucosal, transdermal, intramuscular, subcutaneous or intrarectal administration other than oral or intravenous administration, and depending on the administration route, several preparations may be used. Each preparation is described herein after, but formulation used in this invention is not limited thereto, any kind of formulation used in pharmaceutical preparation may be used.

Used as preventive agent or therapeutical agent for condition associated with protein modification products, an oral dose of the compound (I) or (II) is generally range of preferably 0.3 mg/kg to 300 mg/kg, more preferably 1 mg/kg to 100 mg/kg. In systemic administration, especially in intravenous administration, dose will vary depending on their sex, age, body weight or etc. but usually administer to make available blood level in the range of 2 µg/mL to 200 µg/mL, more preferably 5 µg/mL to 100 µg/mL

Dosage form of oral administration includes powder, granule, capsule, pill, tablet, elixir, suspension, emulsion and syrup, and selects suitably. Further, dosage form of intraorally local administration includes masticatory, sublingual formulation, buccals, lozenge, ointment, adhesive preparation and liquid, and selects suitably. Besides, some modifications such as sustained release, stable, ease disintegrate, hard disintegrate, enteric, ease absorption may be done.

Any known drug delivery system (DDS) may be adopted to each preparations sited above. DDS preparation herein means the most suitable preparation form such as sustained release preparation, topical applicable preparation (such as lozenge, buccals and sublingual formulation), controlled release preparation, enteric preparation and gasteric preparation based on administration route, bioavailability, adverse effect and etc.

The component of DDS includes essentially medicament, medicament release module, encapsulating body and therapeutic program. For each component, in particular, the medicament whose blood level goes down quickly when stopped the release and which has a short half life is preferred, the encapsulating body which does not react with living tissue of administration site is preferred, in addition, the therapeutic program which maintain the best drug level in given period is preferred. The medicament release module comprise essentially medicament container, release controlling part, energy source and release hole or release surface. Such essential component is not needed getting together all of them, thus the best form can be selected by suitable addition or deletion.

Materials for DDS include high molecule, cyclodextrin derivative, and lecithin. The high molecule are selected from insoluble high molecule (such as silicon, ethylene-acetic vinyl copolymer, ethylene-vinylalcohol copolymer, ethylcellulose and cellulose acetate), soluble high molecule and hydroxyl gel forming high molecule (such as polyacrylamide, polyhydroxyethyl methacrylate cross-linked material, polyacryl cross-linked material, polyvinylalcohol, polyethylene oxide, water-soluble cellulose derivative, cross-linked poloxamer, chitin, chitosan), sustained soluble high molecule (such as ethylcellulose, partial ester of methylvinyl ether-maleic acid anhydride copolymer), gastric high molecule (such as hydroxypropylmethyl cellulose, hydroxy propylcellulose, carmellose sodium, macrogol, polyvinyl pyrolidone, methacrylic acid dimethylaminoethyl-methacrylic acid methyl copolymer), enteric high molecule (such as hydroxypropylmethyl cellulose phthalate, acetic acid phthalcellulose, hydroxypropylmethyl cellulose acetate succinate, carboxymethylethyl cellulose, acrylic acid group polymer), biodegradable high molecule (such as thermocoagulation or cross-linked albumin, cross-linked gelatin, collagen, fibrin, polycyanoacrylate, polyglycolic acid, polylactic acid, poly β-hydroxy acetic acid, polycaprolactam)

Particularly, silicon, ethylene-acetic acid vinyl copolymer, ethylenevinylalcohol copolymer and partial ester of methylvinyl ether-maleic acid anhydride copolymer may use for release control of drug, and cellulose acetate may use as a material of osmotic pump, ethylcellulose, hydroxypropylmethyl cellulose, hydroxypropyl cellulose and methylcellulose may use for membrane material of sustained release preparation, polyacryl cross-linked material may use for adhesive preparation for oral or ocular mucosa.

In addition, depending on the formulation (such as formulation for oral administration, injection, suppository), the preparation may be added suitable additives, for example solvent, diluent, coating agent, base, binding agent, lubricant, disintegrant, solubilizing agent, suspending agent, thickner, emulsifier, stabilizer, buffer, tonicity agent, soothing agent, presevative, flavoring agent, aromatic agent and/or colorant. For such additives, examples are listed following after, but not limited to.

The solvent includes purified water, injection solvent, saline, arachis oil, ethanol and glycerin. The diluent includes starch, lactose, glucose, sucrose, crystalline cellulose, calcium sulfate, calcium carbonate, talc, titanic oxide, trehalose and xylitol. The coating agent includes sucrose, gelatin, cellulose acetate phthalate and high molecule as sited above. The base includes petrolatum, vegetable oil, macrogol, oil in water emulsifier base and water in oil emulsifier base.

The binding agent includes starch and derivatives thereof, cellulose and derivative thereof, gelatin, alginate sodium, tragacanth, nature high molecules such as gum acacia, synthetic high molecules such as polyvinyl pyrrolidone, dextrin and hydroxypropyl starch. The lubricant includes stearic acid and salt thereof, talc, wax, wheat starch, macrogol, hydrogenated vegetable oil, sucrose fatty acid ester and polyethylene glycol. The disintegrant includes starch and derivative thereof, agar, gelatin powder, sodium hydrogen carbonate, cellulose and derivative thereof, carmellose calcium, hydroxypropyl starch, carboxymethyl cellulose and salt thereof as well as cross-linked structure there of and lower substituted hydroxypropyl cellulose.

The solubilizing agent includes cyclodextrin, ethanol, propylene glycol and polyethylene glycol. The suspending agent includes gum acacia, tragacanth, alginate sodium, alminum monostearate, citric acid and several surfactants. The thickner includes carmellose sodium, polyvinyl pyrrolidone, methylcellulose, hydroxypropylmethyl cellulose, polyvinylalcohol, tragacanth, gum acacia and alginate sodium. The emulsifier includes gum acacia, cholesterol, tragacanth, methylcellulose, various surfactants and lecithin.

The stabilizer includes bisulfite sodium, ascorbic acid, tocopherol, chelate agent, innert gas and reducing material. The buffer includes hydrogen phosphate sodium, acetic acid sodium and boric acid. The tonicity agent includes sodium chloride and glucose. The soothing agent includes procaine hydrochloride, lidocaine and benzyl alcohol. The preservative includes benzoic acid and salt thereof, p-hydroxybenzoic esters, chloro butanol, cationic soap, benzyl alcohol, phenol and thyromethal. The flavoring agent includes sucrose, saccharine, licorice extract, sorbitol, xylitol and glycerin. The aromatic agent includes spruce tinctura and rose oil. The colorant includes water solubility food colorant and lake dye.

As described above, continuance of available blood level, enhanced bioavailability and etc. may predict by preparing DDS preparation such as sustained release preparation, enteric preparation or controlled drug release preparation. However, the compound (I) or (II) is disactivated or degraded in an organism, and as the result, there is possibility that desired effect may reduce or disappear. Accordingly, material(s) which inhibit the deactivater or degradater of the compound (I) or (II) are combined with the composition for prevention of treatment for the condition associated with protein modification products of this invention to continue the effect of the ingredient. Such material(s) may mix in preparation, and may administer separately. The material(s) which deactivate or degrade the compound (I) or (II) are identified, inhibitor of it such material(s) may be selected, and mixed or used together by a person skilled in the art suitably.

In the preparation, any ingredient used in normal composition as additives other than described above may be used, and an amount of such ingredient is selected in the range that the effect of this invention is not prevented.

The compound (I) or (II) of this invention may be also used to suppress the disorder from protein modification products in peritoneal dialysis and hemodialysis. Namely, the compound (I) or (II) as inhibitor of protein modification products formation is added to conventional peritoneal dialysate or hemodialysate.

The method to reduce an amount of carbonyl compound in liquid sample according to this invention comprises the step that said liquid sample are contacted with the compound (I) or (II) as inhibitor of protein modification products formation.

In addition, the method to suppress the formation of protein modification products according to this invention comprises the step that blood from patient or peritoneal dialysate is contacted with the compound (I) or (II) as inhibitor of protein modification products formation. The protein modification products in dialysis includes protein modification products that is formed by the carbonyl compound derived from patient with peritoneal dialysis or hemodialysis and the protein modification products that is formed by carbonyl compound derived from peritoneal dialysate or hemodialysate themselves.

The composition of peritoneal dialysate or hemodialysate that is added the compound (I) or (II) according to this invention is selected from known one. Common peritoneal dialysate are composed of osmo-regulator (such as glucose), buffer (such as lactose, citric acid, malic acid, acetic acid, pyruvic acid, succinic acid and sodium hydrogencarbonate), and inorganic salts (such as sodium ion, potassium ion, magnesium ion, calcium ion and chloride ion). The peritoneal dialysate or hemodialysate that was added the compound (I) or (II) may be sealed off directly to sterilize by heat. By so doing, the formation of protein modification products from main ingredients accompanied with sterilization by heat or preservation.

In addition, liquid such as peritoneal dialysis is packed in separated container that consists of first chamber and second chamber, reducing sugar is packed in the first chamber and the compound (I) or (II) is packed in the second chamber, and all of them may be mixed before use. When amino acid is comprised, a person skilled in the art may adopt the best constitution such as third chamber.

Since the compound (I) or (II) suppresses the formation of protein modification products by intraperitoneal or intravascular administration, adverse effects such as peritoneal sclerosis may alleviated. Furthermore, it can be expected to work prevention and/or therapy of other conditions (such as diabetic complication). The dialysate may involve known agent such as amino guanidine other than the compound (I) or (II). Alternatively, it can be adopted in powdered dialysis agent.

The compound (I) or (II) may be injected to dialysis circuit that is equipped with suitable connector for coinjection. Alternatively, the compound (I) or (II) is directly injected into peritoneal cavity to mix with peritoneal dialysate in the peritoneal cavity. Alternatively, before peritoneal dialysate is injected to patient or while it collects in a peritoneal cavity, the compound (I) or (II) may be injected intravenously to suppress the formation of protein modification products effectively.

The dialysate is filled in suitable sealing container and sterilized. The sterilization by high-pressure steam and by hot-water is effective. In this case, the container that toxic substances are not eluted at high temperature and that have enough hardness to endure carriage after sterilization is used. In particular, commutative plastic bag that is made from for example polyvinyl chloride, polypropylene, polyethylene, polyester, ethylene acetate vinyl copolymer are included. In addition, to avoid degradation of liquid due to the effect of ambient air, the container that is filled the dialysate are further packed by packing materials which has high gas barrier property.

When sterilization is carried out by heat including high-pressure heat, if the compound (I) or (II) used has enough stability against treatment such as heat, the compound (I) or (II) is added previously to dialysate and then the mixed dialysate is sterilized. If the compound (I) or (II) used does not have stability against sterilization by heat, sterilization without heat can be carried out. Such sterilization includes, for example sterilization by filtration.

For example, such sterilization can be carried out by filtration with fine filter that is equipped with membrane filter having pore diameter about 0.2 µm. The dialysate that is sterilized by filtration is filled in the container such as flexible plastic bag, and then it is sealed. In addition, to peritoneal dialysate that is previously sterilized by heat may add the compound (I) or (II).

The timing of addition is not limited. Whether after or before sterilization, the compound (I) or (II) may be added, may be added just before or together with dialysis and may inject into peritoneal cavity directly after dialysate is injected.

The peritoneal dialysate of this invention is used for dialysis as current peritoneal dialysate or hemodialysate. Namely, for peritoneal dialysis, suitable amount of the peritoneal dialysate according to this invention is injected into peritoneal cavity of patient with dialysis to transfer low molecular weight ingredient in an organism into peritoneal dialysate through peritonea. The peritoneal dialysate is intermittently circulated and dialysis is continued depending on the condition of patient. At this stage, the compound (I) or (II) suppresses the formation of protein modification products in the dialysate or an organism. The carbonyl compounds transfer from blood or intraperitonea to peritoneal dialysate together with dialysis ingredient such as creatinine, inorganic salts or chlorine ion. Accordingly, adverse effect on an organism by protein modification products is reduced.

The compound (I) or (II) is used for not only dialysate, but also any liquid medicament such as nutrient infusion, electrolyte infusion or enteral or tube feeding.

### EXMPLES

This invention will be hereinafter illustrated more in details by way of Examples, but the scope of this invention should not be understood to be limited to these Examples.

### Preparation Example 1

### Preparation of 4-(5-phenyltetrazol-1-ylmethyl)-morpholine (Compound 1)

To a solution of 26.0 g (0.18 mol) of 5-phenyltetrazole in 270 ml of methanol, were added 17.0 g (0.2 mol) of morpholine and 17.8 g of 36% formalin at 5°C, followed by stirring at room temperature overnight. To the reaction mixture, hexane (200 ml) was added, and the precipitated crystals were collected by filtration. The collected crystals were dried in vacuum to give 4-(5-phenyltetrazol-1-ylmethyl)-morpholine (43.6 g; yield, 99%) as white crystals. The structure was confirmed by nuclear magnetic resonance spectrum (NMR) and mass spectrum (Mass)
NMR: significant signals
2.714ppm t 4H morpholineCH2
3.709ppm t 4H morpholineCH2
5.499ppm s 2H CH2 between tetrazole and morpholine
7.494ppm m 3H phenyl CH
8.171ppm m 2H phenyl CH
Mass(EI-MS): m/z 245 (molecular weight)

In the same manner, 5-methyl-1-(5-phenyltetrazol-1-ylmethyl)-3a,6a-dihydro-1H-pyrrolo[3,4-d][1,2,3]triazole-4,6-dione (Compound 2) and (5-phenyltetrazol-1-yl)-methanol (Compound 3) were synthesized. These are known compounds.

### Preparation Example 2

### Preparation of 1-(5-phenyltetrazol-1-ylmethyl)-piperazine (Compound 4)

5-Phenyltetrazole (2.00 g; 13.8 mmol) was dissolved in 20 ml of methanol, and 2.37 g (27.6 mmol) of piperazine and 1.342 g (16.54 mmol) of 36% formalin were added thereto, followed by stirring at a temperature below 10°C and warming to room temperature. This mixture was stirred overnight and reacted. The objective compound formed with the progress of the reaction was precipitated as crystals, because of its highly poor solubility. After completion of the reaction, the precipitated crystals were collected by filtration and dried in a desiccator in vacuum to give 0.959 g of 1-(5-phenyltetrazol-1-ylmethyl)-piperazine as white crystals.

### Preparation Example 3

### Preparation of 1-methyl-4-(5-phenyltetrazol-1-ylmethyl)-piperazine (Compound 5)

5-Phenyltetrazole (13.0 g; 89.6 mmol) was added in 113 ml of methanol and cooled, and 9.82 g (98.0 mmol) of methylpiperazine and 9.2 g (0.11 mol) of 36% formalin were added thereto, followed by stirring. The mixture was then stirred at room temperature overnight to react. After removal of methanol by distillation, the residue was suspended in water, and the precipitated crystals were collected by filtration. The collected crystals were washed with water and dried to obtain 13.0 g (yield 56.2%) of 1-methyl-4-(5-phenyltetrazol-1-ylmethyl)-piperazine as beige crystals.

The structure of 1-methyl-4-(5-phenyltetrazol-1-ylmethyl)-piperazine was confirmed by nuclear magnetic resonance spectrum (NMR). The result supported that the structure of the objective compound. The data is as shown below.
NMR: significant signal
2.258ppm s 3H CH3
2.455ppm t 4H piperazine CH2
3.761 ppm t 4H piperazine CH2
5.521 ppm s 2H CH2 between tetrazole and piperazine
7.480ppm m 3H phenyl CH
8.125ppm m 2H phenyl CH

### Preparation Example 4

### Preparation of 4-(5-phenyltetrazol-1-ylmethyl)-thiomorpholine (Compound 6)

5-Phenyltetrazole (13.0 g; 0.089 mol) was dissolved in 133ml of methanol and cooled at a temperature below 5°C. To the resultant solution, 10.0 g (0.10 mol) of thiomorpholine and 9.2g of 36% formalin were added, followed by stirring at room temperature overnight. After completion of the reaction, the objective compound precipitated as crystals was collected by filtration and dried in a vacuum desiccator to obtain 11.5 g (yield 49.1%) of 4-(5-phenyltetrazol-1-ylmethyl)-thiomorpholine as white crystals.

The structure of 4-(5-phenyltetrazol-1-ylmethyl)-thiomorpholine was confirmed by nuclear magnetic resonance spectrum (NMR). The result supported the structure of the objective compound. The data is as shown below.
NMR: significant signal
2.685ppm t 4H thiomorpholine CH2
2.988ppm t 4H thiomorpholine CH2
5.480ppm s 2H CH2 between tetrazole and thiomorpholine
7.494ppm m 3H phenyl CH
8.162ppm m 2H phenyl CH

### Preparation Example 5

### Preparation of 1-4-bis-(5-phenyltetrazol-1-ylmethyl)-piperazine (Compound 7)

To the solution of 20.0 g (0.14 mol) 5-phenyltetrazole in 205 ml of methanol, were added 5.3 g (0.062 mol) of piperazine and 14.1 g of 36% formalin at the temperature below 5°C, followed by stirring at room temperature overnight. After completion of the reaction, the precipitated crystals were collected by filtration and dried in a vacuum desiccator to obtain 23.7 g (yield 95.0%) of 1,4-bis-(5-phenyltetrazol-1-ylmethyl)-piperazine as white crystals.

The structure of 1,4-bis-(5-phenyltetrazol-1-ylmethyl)-piperazine was confirmed by nuclear magnetic resonance spectrum (NMR). The result supported the structure of the objective compound. The data is as shown below.
NMR: significant signal
2.767ppm s 8H piperazine CH2
5.469ppm s 4H CH2 between tetrazole and piperazine
7.496ppm m 6H phenyl CH
8.171ppm m 2H phenyl CH

### Test Example 1

### Examination of the AGEs formation inhibiting effect

For 4-(5-phenyltetrazol-1-ylmethyl)-morpholine (Compound 1), 5-methyl-1-(5-phenyltetrazol-1-ylmethyl)-3a,6a-dihydro1 H-pyrrolo[3,4-d][1,2,3]triazole-4,6-dione (Compound 2) and (5-phenyltetrazol-1-yl)methano (Compound 3), the AGEs formation inhibiting effect was examined by measuring an amount of pentosidine as a typical example of AGEs according to the procedure as described below.

Before dialysis, blood samples were obtained from non-diabetic volunteer dialysis patients with renal failure, and fresh heparinized plasma samples were prepared therefrom. Pooled plasma obtained from several patients (n = 3~5) was served to the test. 4-(5-phenyltetrazol-1-ylmethyl)-morpholine (Compound 1), 5-methyl-1-(5-phenyltetrazol-1-ylmethyl)-3a,6a-dihydro-1H-pyrrolo[3,4-d][1,2,3]triazole-4,6-dione (Compound 2) or (5-phenyltetrazol-1-yl)methanol (Compound 3) adjusted to a designed concentration (final concentration, 8, 20 or 50mM), in DMSO solution (100µl), was added to the pooled plasma (900µl) and incubated at 37°C for 7 days in the presence of air. Then, the amount of pentosidine was measured to estimate the suppressive effect on the glycation of protein.

Measurement of the amount of pentosidine was made as follows: to each incubated sample (50µL) was added an equal volume of 10% trichloroacetic acid, followed by centrifugation at 5000 g for 5 minutes; after removal of the supernatant, the pellet was washed by 5% trichloroacetic acid (300µL); the pellet was dried under reduced pressure and then subjected to hydrolysis in 6N HCl solution (100µL) at 110°C under nitrogen atmosphere for 16 hours; to the hydrolysate, 5N NaOH (100 µL) and 0.5 M phosphate buffer (pH 7.4) (200 µL) were added, followed by filtration through a porefilter with a pore size of 0.5 µm and dilution with PBS. The concentration of free pentosidine was determined by reversed-phase HPLC using a fluorescence detector (RF-10A, Shimazu Seisakusho) (Miyata, T. et al.: Proc. Natl. Acad. Sci. USA, 93, 2353-2358, 1996). The effluent was monitored at 335/385 nm of excitation/emission wavelength. Synthetic pentosidine was used as the standard. The detection limit of pentosidine was 0.1 pmol/mg protein.

The inhibiting effect was estimated by comparing with positive control (aminoguanidine and pyridoxamine (Sigma)) reacted in the same manner as 4-(5-phenyltetrazol-1-yl-methyl)-morpholine (Compound 1), 5-methyl-1-(5-phenyltetrazol-1-ylmethyl)-3a,6a-dihydro1H-pyrrolo[3,4-d][1,2,3]triazole-4,6-dione (Compound 2) and (5-phenyltetrazol-1-yl)methanol (Compound 3).

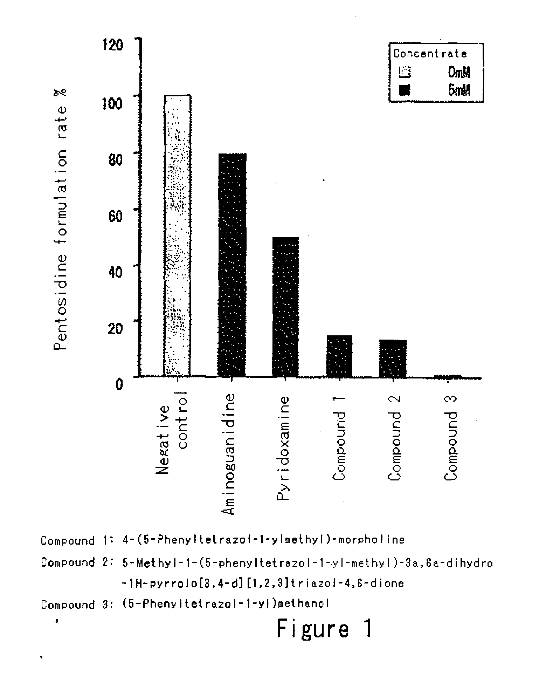

The pentosidine formation inhibiting effects (formed pentosidine amount, nmol/mL) and the pentosidine formation rates (%) of 4-(5-phenyltetrazol-1-ylmethyl)-morpholine, 5-methyl-1-(5-phenyltetrazol-1-yl methyl)-3a,6a-dihydro-1H-pyrrolo[3,4-d][1,2,3]triazole-4,6-dione and (5-phenyltetrazol-1-yl)methanol when incubated with plasma from the patients with dialysis are respectively shown in Table 2 and Figure 1.

| Table 2 | | |
|---|---|---|
| Test compound | Concentration | Pentosidine formation (nmol/mL) |
| DMSO (negative control) | | 2.960 |
| Aminoguanidine (positive control) | 5mM | 2.604 |
| Pyridoxamine (positive control) | 5mM | 2.280 |
| 4-(5-Phenyltetrazol-1-ylmethyl)-morpholine (Compound 1) | 5mM 5mM 0.367 | 0.367 |
| 5-Methyl-1-(5-phenyltetrazol-1-yl-methyl)-3a,6a-dihydro-1H-pyrrolo[3,4-d][1,2,3]triazol-4,6-dione (Compound 2) | 5mM | 0.320 |
| (5-Phenyltetrazol-1-yl)methanol (Compound 3) | 5mM | 0.026 |

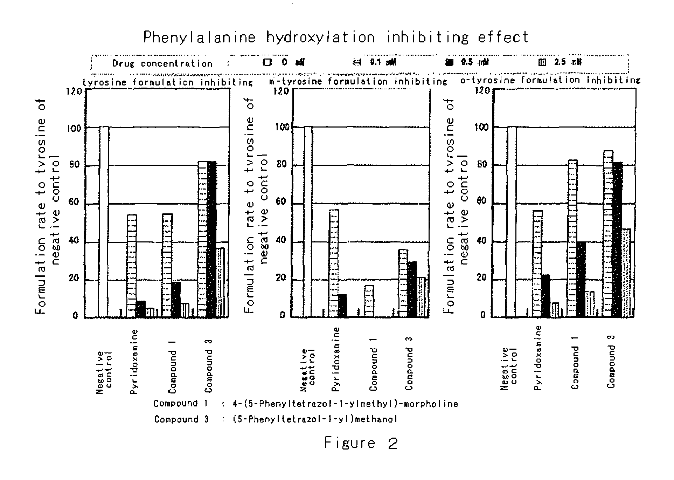

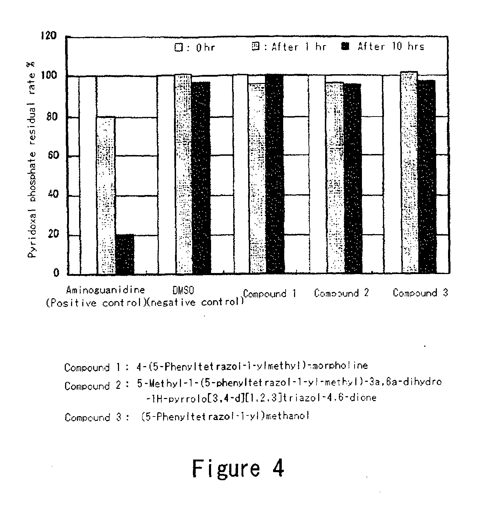

As shown in Table 2 and Figure 2, 4-(5-phenyltetrazol-1-ylmethyl)-morpholine (Compound 1), 5-methyl-1-(5-phenyltetrazol-1-ylmethyl)-3a,6a-dihydro-1H-pyrrolo[3,4-d] [1,2,3]triazole-4,6-dione (Compound 2) and (5-phenyltetrazol-1-yl)methanol (Compound 3) suppressed remarkably the pentosidine formation in the plasma from the patients with dialysis.

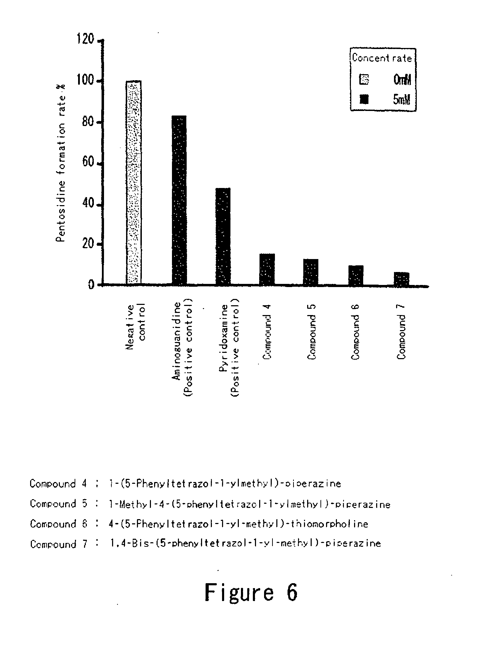

In the same manner as above, the pentosidine formation inhibitory activity of 1-(5-phenyltetrazol-1-ylmethyl)-piperazine (Compound 4), 1-methyl-4-(5-phenyltetrazol-1-ylmethyl)-piperazine (Compound 5), 4-(5-phenyltetrazol-1-ylmethyl)-thiomorpholine (Compound 6) and 1,4-bis-(5-phenyltetrazol-1-ylmethyl)-piperazine (Compound 7) was determined (cf. Table 3 and Figure 6). Those compounds suppressed remarkably the pentosidine formation.

| Table 3 | | |
|---|---|---|
| Test compound | Concentration | Pentosidine formation |
| 1-(5-Phenyltetrazol-1-ylmethyl)-piperazine (Compound 4) | 5mM | 0.234 nmol/mL |
| 1-Methyl-4-(5-phenyltetrazol-1-ylmethyl)-piperazine (Compound 5) | 5mM | 0.201 nmol/mL |
| 4-(5-Phenyltetrazol-1-yl-methyl)-thiomorpholine (Compound 6) | 5mM | 0.149 nmol/mL |
| 1,4-Bis-(5-phenyltetrazol-1-yl-methyl)-piperazine (Compound 7) | 5mM | 0.103 nmol/mL |

### Test Example 2

### (1) The suppressive effect on the hydroxylation of phenylalanine with hydroxy radical

For 4-(5-phenyltetrazol-1-ylmethyl)-morpholine (Compound 1), 5-methyl-1-(5-phenyltetrazol-1-ylmethyl)-3a,6a-dihydro-1H-pyrrolo[3,4-d][1,2,3]triazole-4,6-dione (Compound 2) and (5-phenyltetrazol-1-yl)methanol (Compound 3), the suppressive effect on the hydroxylation of phenylalanine with hydroxyl radical was examined.

Phenylalanine (final concentration: 1 mM), test compound (final concentration: 0.1, 0.5, 2.5 mM), hydrogen peroxide (final concentration: 5mM) and cupric sulfate (final concentration: 0.1mM) were dissolved in 200 mM of phosphate buffer (pH7.4) (total volume 500 µL), followed by incubation at 37°C for 4 hours. Then, DTPA (final concent-ration: 1mM) and 260 unit of catalase were added thereto to interrupt the reaction. The amounts of o-tyrosine and m-tyrosine formed were determined by HPLC in the following manner: after a designed period of time, the reaction mixture was diluted to 100 folds; 20µL of the dilution was injected onto HPLC, separation was made with C18 column (4.6 x 250 mm, 5µm; Nomura Kagaku) and detection was effected using a fluorescence detector (RF-10A: Shimazu Seisakusho) under the condition of an excitation wavelength of 275nm and a fluorescence wavelength of 305nm. In the mobile phase, the flow rate was 0.6mL/min and the concentration of buffer B was varied from 6.5% to 10% in 25 minutes (buffer A: 0.10% trifluoroacetic acid; buffer B: 80% acetonitrile containing 0.08% trifluoroacetic acid). The result is shown in Figure 2.

4-(5-Phenyltetrazol-1-ylmethyl)-morpholine (Compound 1), 5-methyl-1-(5-phenyltetrazol-1-ylmethyl)-3a,6a-dihydro 1H-pyrrolo[3,4-d][1,2,3]triazol-4,6-dione (Compound 2) and (5-phenyltetrazol-1-yl)methanol (Compound 3) suppressed remarkably the hydroxylation of phenylalanine with hydroxy radical in comparison with pyridoxamine (Sigma) as the positive control.

### (2) The suppressing effect on the nitration of tyrosine with peroxynitrite

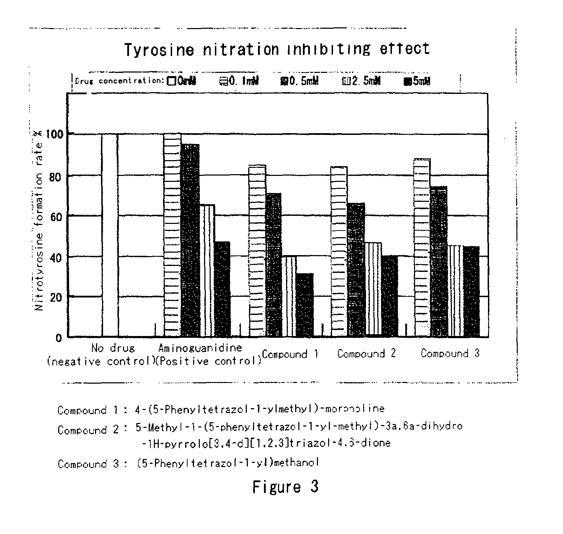

According to the method of Pannala, A.S. et al. (Free Radic. Biol. Med., 24:594-606, 1998), the examination was carried out. Namely, tyrosine (final concentration: 100 µM), the test compound (final concentration: 0.1, 0.5, 2.5 and 5mM) and peroxynitrite (Dojin Kagaku) (final concentration: 500µM) were dissolved in 200mM of phosphate buffer (pH7.4) (liquid volume 500µL) and incubated at 37°C for 15 minutes. After incubation, the nitrotyrosine formation was determined with HPLC in the following manner: after a predifined time, the reaction mixture (20µL) was injected onto HPLC, separation was made with C18 column (4.6 x 250 mm, 5 µm: Waters) and the detection was effected using a ultraviolet detector (RF-10A: Shimazu Seisakusho) at a wavelength of 280nm. In the mobile phase, the flow rate was 0.6 mL/min and the concentration of buffer B was varied from 5.0% to 30% in 30 minutes (buffer A: 0.10% trifluoroacetic acid; buffer B: 80% acetonitrile containing 0.08% trifluoroacetic acid). 4-Hydroxy-3-nitrobenzoic acid (100µM) was used as the internal standard. The result is shown in Figure 3.

4-(5-Phenyltetrazol-1-ylmethyl)-morpholine (Compound 1), 5-methyl-1-(5=phenyltetrazol-1-ylmethyl)-3a,6a-dihydro-1H-pyrrolo[3,4-d][1,2,3]triazol-4,6-dione (Compound 2) and (5-phenyltetrazol-1-yl)methanol (Compound 3) suppressed remarkably the tyrosine formation by nitration of tyrosine in comparison with pyridoxamine (Sigma) as the positive control.

### Test Example 3

### Examination of vitamin B6 (PLP; pyridoxal phosphate) trapping

4-(5-Phenyltetrazol-1-ylmethyl)-morpholine (Compound 1), 5-methyl-1-(5-phenyltetrazol-1-ylmethyl)-3a,6a-dihydro-1H-pyrrolo[3,4-d][1,2,3]triazol-4,6-dione (Compound 2) and (5-phenyltetrazol-1-yl)methanol (Compound 3) as well as aminoguanidine as the positive control were each dissolved in DMSO in a designed concentration to make a sample solution. DMSO itself was used as the negative control. In addition, pyridoxal phosphate (PLP) was dissolved in purified-water to make a designed concentration. PBS was added to each of the test compound, the positive control and the negative control, to make a final concentration of 500 µM. The PLP solution as above prepared was added to each of the resultant solution to make a final concentration of 50 µM for preparation of the PLP reaction solution. Using HPLC, the amount of PLP in the PLP reaction solution was determined after 0, 1 and 10 hours, from which the PLP trapping amount of each test compound was ascertained.

The analysis of PLP using HPLC was carried out by separation with reverse-phase C18 column (4.6 x 250 mm, 5 *µ*m; Waters) and by using a fluorescence detector (RF-10A; Shimazu Seisakusho; excitation wavelength, 300 nm and fluorescence wavelength, 400nm). In the mobile phase, the flow rate was 0.6 mL/min and the concentration of buffer B was varied from 0% to 3% in 25 minutes (buffer A: 0.10% trifluoroacetic acid; buffer B: 80% acetonitrile containing 0.08% trifluoroacetic acid). The PLP residual rate is shown in Figure 4.

As the results, aminoguanidine as the positive control showed remarkable PLP trapping but none of 4-(5-phenyltetrazol-1-ylmethyl)-morpholine (Compound 1), 5-methyl-1-(5-phenyltetrazol-1-ylmethyl)-3a,6a-dihydro-1 H-pyrrolo[3,4-d][1,2,3]triazole-4,6-dione (compound 2) and (5-phenyltetrazol-1-yl)methanol (Compound 3) showed PLP trapping.

### Test Example 4

### Absorbability examination

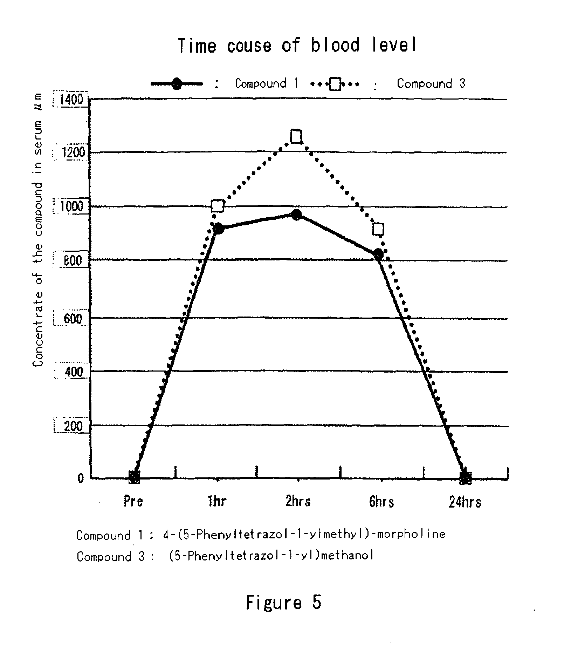

For 4-(5-phenyltetrazol-1-yl methyl)-morpholine (compound 1) and (5-phenyltetrazol-1-yl) methanol (compound 3), their sorbability were examined. Each compounds are suspended in carboxymethyl cellulose in given concentration to prepare sample for oral administration. Then each samples were administered in the ratio of 50 mg/kg to rat using sonde. Five Wister rats (male, 8 weeks aged, closed colony) were used every one group. At one hour, 2 hours, 6 hours and 24 hours after the administration of compound, blood was obtained. The obtained blood samples were immediately cetrifuged at 3000 rpm for 15 minutes to obtain the blood plasma and the concentration of the compound in the specimen was quantitated by HPLC. Briefly, to 100µL of obtained blood plasma was added to 200µL of acetonitrile, centrifuged at 12000 rpm for 10 minutes to obtain supernatant, deproteinized and served to HPLC. The measuring condition of HPLC was selected from suitable condition for each compounds to separate and quantify. For example, in case of 4-(5-phenyltetrazol-1-yl methyl)-morpholine (compound 1), separated with reverse-phase C18 column (4.6 x 250 mm, 5 µm: Waters), and then detected using ultraviolet detector (RF-10A: Shimazu Seisakusho) at 240 nm of wavelength to quantify. In mobile phase, the flow rate is 0.8 mL/min and solvent is water: acetonitrile. The result is shown in Figure 5. Every compounds indicate good sorbability.

### Test Example 5

### Acute toxicity examination

For 4-(5-phenyltetrazol-1-ylmethyl)-morpholine (compound 1) and (5-phenyltetrazol-1-yl) methanol (compound 3), acute toxicity test was carried out using mice. Each compounds were suspended in carboxymethyl cellulose in given concentration to prepare sample for oral administration. Then single oral administration was done to mouse. The dose is 100, 250, 500, 1000 and 2000 mg/kg. Five ICR mice (male , 8 weeks aged, closed colony) were used in each group. The test material was administrated to determine LD₅₀ value. The result is indicated in Table 4. Every compound has lower toxicity.

| Table 4 | |
|---|---|
| Compound | LD₅₀ |
| 4-(5-phenyl-tetrazol-1-yl methyl)-morpholine (compound 1) | >1000mg/kg |
| (5-Phenyl-tetrazol-1-yl)methanol (compound 3) | >2000mg/kg |

### Test Example 6

### Effect against glomerulonephritis

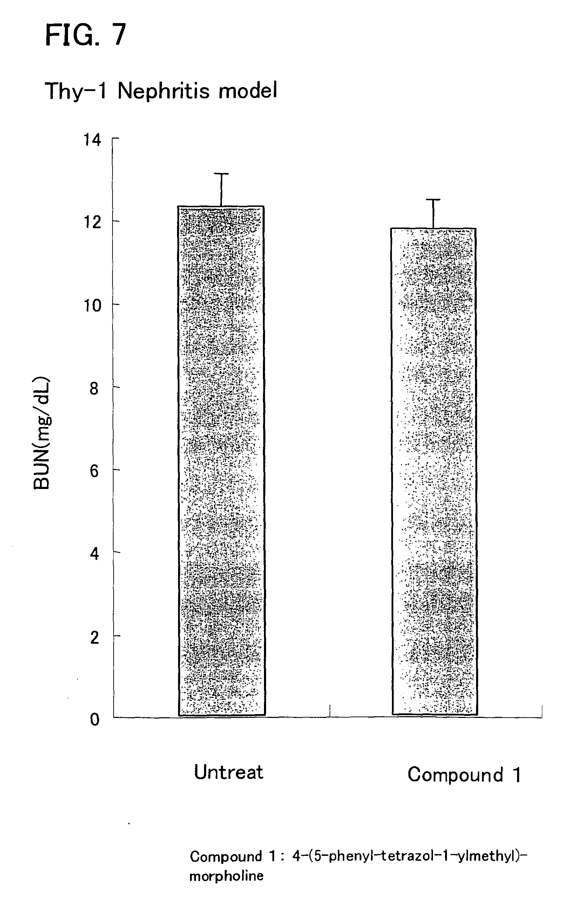

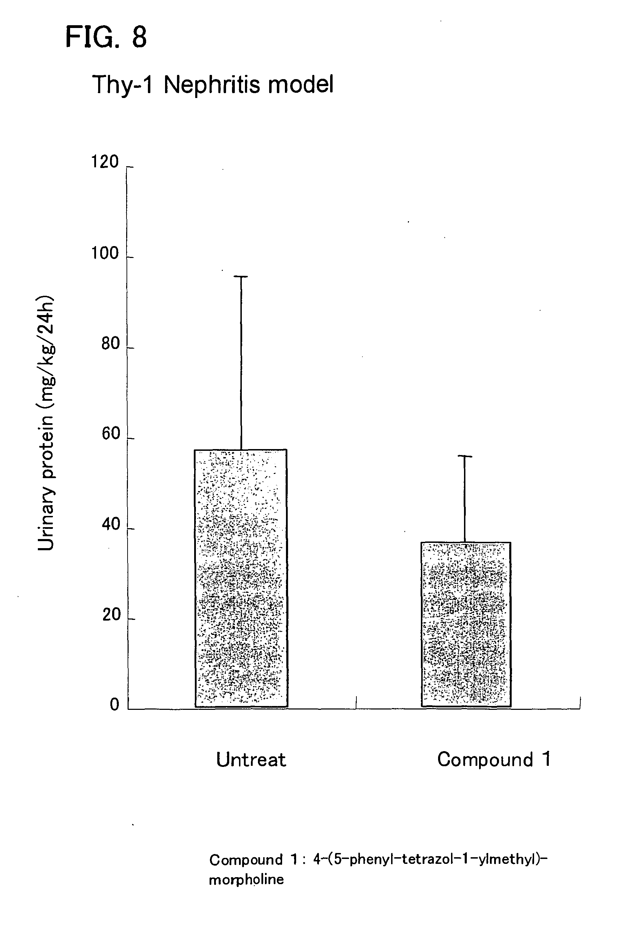

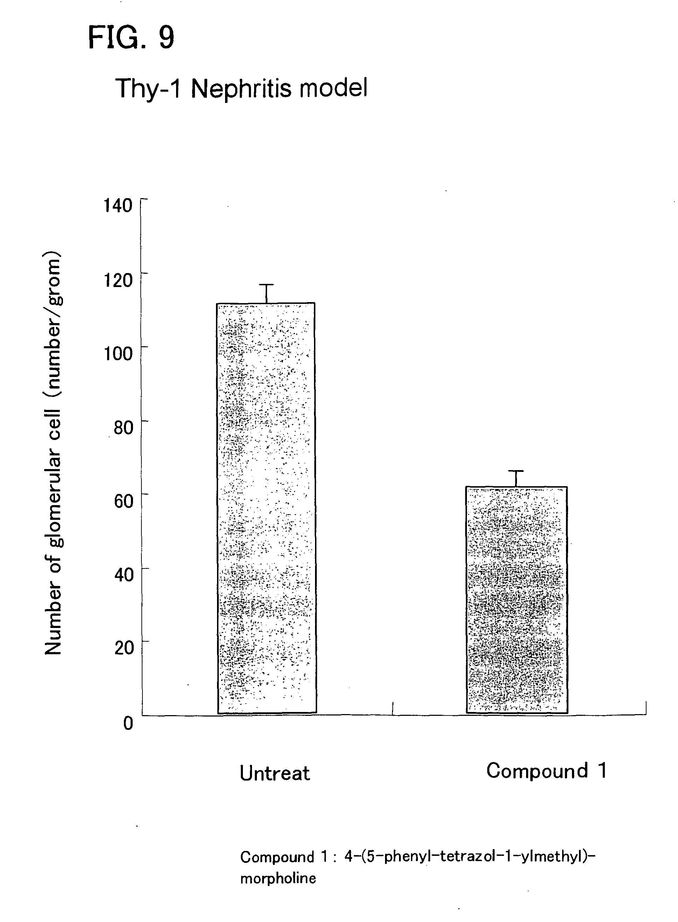

1.2 mg/kg of OX-7, anti Thy-1 antibody, was administered to wistar rat (male, body weight 150 g, 6 weeks old) in tail vein to prepare typical glomerulonephritis model with mesangial proliferative nephritis. After the administration of anti Thy-1 antibody, test compound (4-(5-phenyltetrazol-1-yl methyl)-morpholine (compound 1), 1 mg/kg body weight, once a day) was suspended in 0.5% carboxymethylcellulose, was coercively administered for 5 days continuously, on sixth day sampled, kidney was obtained, and analysed pathologically (count the number of glomerular cells). In particular, cells were stained by PAS according to conventional method, the stained image was captured by 3CCD camera (Olympus), and then analyzed using softwares, Image Graver PCI (FUJI shashin film) and Mac Aspect (Mitani kabushiki kaisha). Also, biochemical analysis of blood and urine was carried out (contract clinical analysis organization: SRL). The results are indicated in Figure 7 to 9.

### Test Example 7

### Renal protective effect on ischemia-reperfusion acute renal failure model rat

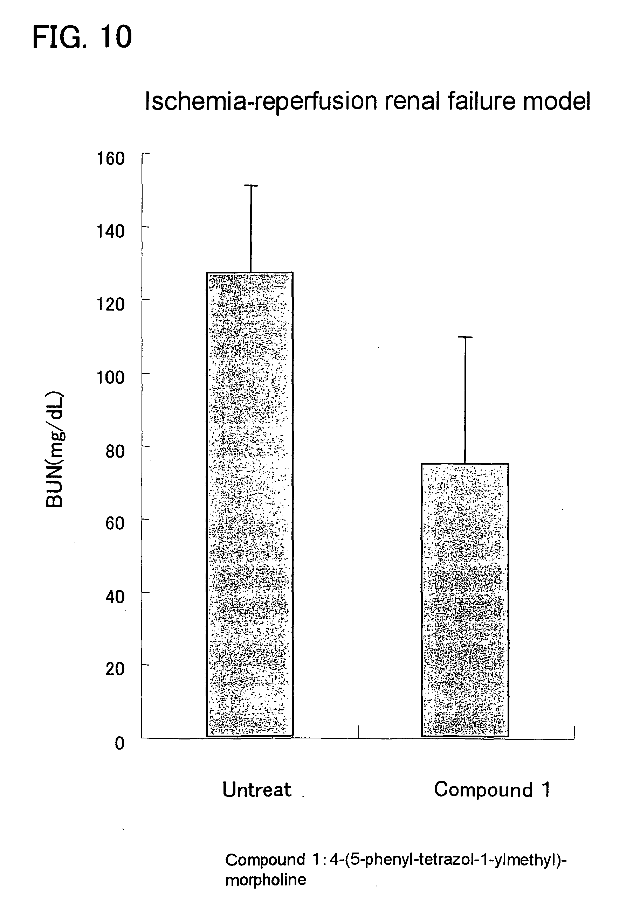

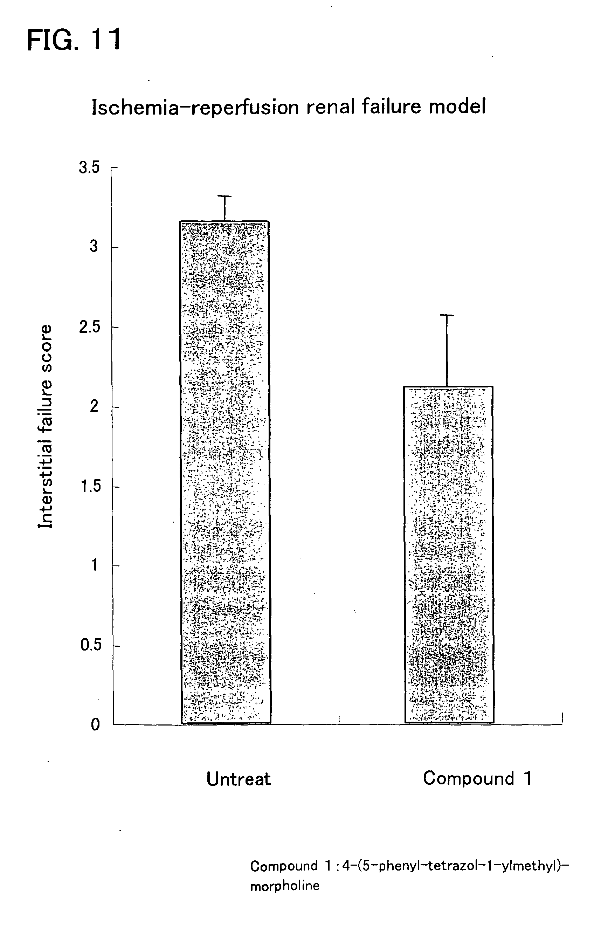

This condition model is typical acute renal failure model. To prepare the model, operated on wistar rat (male, body weight 150 g, 6 weeks aged) to remove right kidney, and on the following day, renal artery of remaining left kidney was applied a ligature with clip under general anesthesia. After the clipping, the rat was on warming plate not to reduce the body temperature and was observed for 45 minutes (ischemia), and then removes the clip to allow re-perfusion. After preparing the ischemia-reperfusion model, test compound (4-(5-phenyltetrazol-1-yl methyl)-morpholine (compound 1), 1 mg/kg body weight, once a day) was suspended in 0.5% carboxymethylcellulose. The suspension was coercively administered 2 days continuously, on third day kidney was obtained, and analysed pathologically (renal tubular stromal disorder score). In particular, the kidney was stained by PAS according to conventional method and the renal tubular stromal disorder was estimated for the presence or absence of renal tubular necrosis, renal tubular hypertrophy, renal tubular atrophia, renal tubular basal lamina thickening and cast in the sustained image of kidney. As well as biochemical analysis of blood was carried out (contract clinical analysis organization: SRL). The results were indicated in Figures 10 and 11.

### Test Example 8

### Cerebroprotective action in middle cerebral artery ischemia-reperfusion model

CD(SD)IGS male rats (Japan Charles River Kabushiki kaisha, Hino farm, room No.22, specific product) (8 rats per one group) having body weight 270 to 350 g were anesthetized by 2% isoflurane (mixed bas comprising of 70% N₂O (laughter gas) and 30% O₂) to immobilize, and then the rats were put on warming plate to keep the rectal and brain temperature 37 to 38 °C. After that, to observe the stability in the examination, canula which was made by polyethylene (PE-50, Becton Dickinson) was inserted and left in caudal artery of said animal, and this made blood drawing and blood pressure determining to monitor the biochemical parameter such as blood sugar level, hematocrit, CO₂ concentration, oxygen partial pressure, pH, blood pressure and etc. In addition, cerebral blood flow in cortex was determined by laser doppler fluorometry (Neuroscience.inc: OMEGA FLOW (FLO-C1)), putting the detection site directly on cranium on the point left 4mm from bregma. Left neck of such animal was incised, and from internal and external carotid fork of common carotid artery to upstream of internal carotid, nylon surgical thread (length 16 mm, diameter 0.2 to 0.3 mm, with silicon coating on its tip 3 mm) was passed and left, middle cerebral artery was obstructed for 2 hours. After that, the thread was removed to release the middle cerebral artery, and blood was reperfused for 21 hours. To each animals, 3.0 mg/kg of 3-methyl-1-phenyl 2-pyrazolin-5-one (control, hereinafter referring to edaravone) and 4.23 mg/kg of 4-(5-phenyltetrazol-1-yl methyl)-morpholine (compound 1) (test compound, hereinafter referring to TM-3001) were respectively administered twice by cannula which is left in caudal artery 5 minutes and 5 hours after middle cerebral artery occlusion. After said operation, the brain was removed from said animal, and after preparing 7 brain slices with 2 mm thickness, to TTC stain (0.8 g of 2,3,5-triphenyltetrazolium chloride (Sigma) dissolved in 40 ml of saline) were soaked at 37°C for 15 minutes to stain, and fixed by 10% neutral formalin liquid to prepare specimen. Such specimens were created the image by CCD camera respectively, and analysed according to method of Swanson et al. (J Cereb Blood Flow Metab 10:290-293; 1994). As the result, cerebral infarction nest was significantly reduced by control agent edaravone and test agent TM-3001 comparing to diluent single administration. The result is indicated in Figure 13. In addition, nervous condition was estimated in the operated rat on horizontal table by grading system according to the method of Bederson et al. (Stroke 17:472-476, 1990) by 4 grades: Grade 0, when push from the side they walk normally without palsy; Grade 1, when push from the side they resist and walk straight to the front with forelimb flexion; Grade 2, when push from the side they do not resist and walk straight to the front; Grade 3, when push from the side they do not resist and cannot walk straight (spin or fall). Furthermore, function recovering was estimated before and after the operation by rotor rod test that estimates how much they can walk on rotating rotor. As the result, improvement for nervous condition and recovering the function were remarkably shown in edaravone (control drug) and TM-3001 (test compound) comparing to diluent single administration. The result is indicated in Table 5.

**Table 5**

| Test Name | | Diluent single administration | | Edaravone | | TM-3001 | |
|---|---|---|---|---|---|---|---|
| | | Ave | SD | Ave | SD | Ave | SD |
| Nervous condition estimation test* | | | | | | | |
| after occlusion | 10 min | 2.9 | 0.3 | 2.8 | 0.4 | 2.4 | 1.1 |
| after occlusion | 2 hrs | 2.9 | 0.3 | 2.7 | 0.5 | 2.9 | 0.4 |
| after reperfusion | 10 min | 2.4 | 1.1 | 2.2 | 1.0 | 2.6 | 0.5 |
| after reperfusion | 3 hrs | 2.3 | 1.1 | 2.1 | 1.0 | 2.4 | 0.5 |
| after reperfusion | 4 hrs 4 hrs | 2.3 | 1.1 | 2.0 | 1.2 | 2.5 | 0.5 |
| after reperfusion | 22 hrs | 1.6 | 1.4 | 1.5 | 1.2 | 1.8 | 1.3 |
| | | | | | | | |
| rotor rod test** | | | | | | | |
| before occlusion | | 202.7 | 123.0 | 197.0 | 123.4 | 300.0 | 0.0 |
| reperfusion | | 67.1 | 70.5 | 153.1 | 132.5 | 64.8 | 116.3 |
| ratio to before occlusion (%) | | 81.4 | 131.8 | 160.4 | 190.0 | 21.6 | 38.8 |

| | | | | | | | |
|---|---|---|---|---|---|---|---|
| *: Numerics in nervous condition estimation test is calculated by grading system of Bederson et al. | | | | | | | |
| **: Numerics in rotor rod test is reading value of count from rotor rod machine. | | | | | | | |
| Abbreviations: min: minute(s), hrs: hours, Ave: average, and SD: standard deviation | | | | | | | |

### INDUSTRIAL APPLICABILITY

This invention provides inhibitor of protein modification products formation which has potent and excellent inhibiting effect of protein modification products formation without reducing blood pressure, and whose active ingredient is the compound having several substituents on tetrazole ring through methylene, especially the compound (I) or (II). This inhibitor of protein modification products formation is available to prevent and/or treat for disease associated with AGEs or ALEs, in particular, can be used as renal tissue protectant alone or together with peritoneal- or hemodialysate.

### BRIEF DESCRIPTION OF THE DRAWINGS

Figure 1 is a chart showing the pentosidine suppressing effect of the compound of the invention.
Figure 2 is a chart showing the suppressing effect of the compound of the invention on the hydroxylation of phenylalanine by hydroxy radical.
Figure 3 is a chart showing the suppressing effect of the compound of the invention on the nitration of tyrosine by peroxy nitrite.
Figure 4 is a chart showing the vitamin B6 trapping ability of the compound of the invention.
Figure 5 is a chart showing the absorbability of the compound of the invention.
Figure 6 is a chart showing the pentosidine formation inhibiting effect of Compounds 4 to 7.
Figure 7 is a chart showing the BUN reducing effect of Compound 1 in glomerulonephritis model.
Figure 8 is a chart showing the urinary protein reducing effect of Compound 1 in glomerulonephritis model.
Figure 9 is a chart showing the influence of Compound 1 on glomerular cells in glomerulonephritis model.
Figure 10 is a chart showing the BUN reducing effect of Compound 1 in acute renal failure model.
Figure 11 is a chart showing the stromal disorder score of Compound 1 in acute renal failure model.

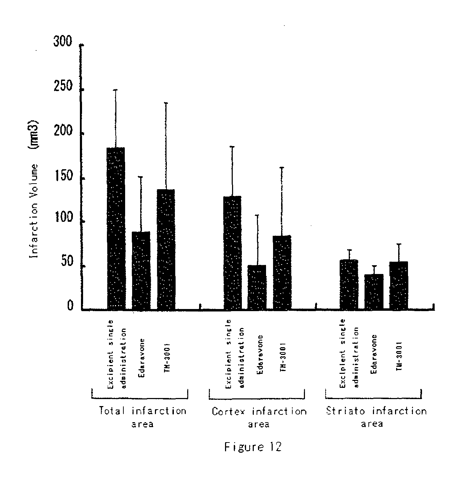

Figure 12 is a chart showing the reduction of the cerebral infarction nest with Compound 1.

## Claims

1. A inhibitor of protein modification products formation comprising a 5-substituted tetrazole ring compound having a methylene-containing group at the 1- or 3-position of the terazole ring in free or salt form as an active ingredient.

2. The inhibitor of protein modification products formation according to claim 1, wherein the compound as the active ingredient is selected from compounds of formula (I); 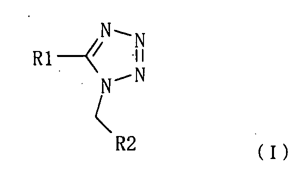 [wherein R1 and R2 represent each the same or different monovalent organic group] in free or salt form.

3. The inhibitor of protein modification products formation according to clam 1, wherein the compound as the active ingredient is selected from compounds of formula (II); 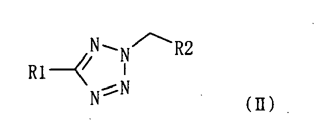 [wherein R1 and R2 represent each the same or different monovalent organic group] in free or salt form.

4. The inhibitor of protein modification products formation according to clam 2 or 3, wherein R1 is an optionally substituted phenyl group and R2 is an optionally substituted heterocyclic group of not more than 10 ring atoms.

5. The inhibitor of protein modification products formation according to clam 2 or 3, wherein R1 is an optionally substituted phenyl group and R2 is a lower alkyl group, a lower alkoxy group or a hydroxy group.

6. The inhibitor of protein modification products formation according to clam 2 or 3, wherein R1 is substituted or unsaturated phenyl group and R2 is morpholino group.

7. The inhibitor of protein modification products formation according to clam 2 or 3, wherein R1 is a substituted or unsubstituted phenyl group and R2 is a 5-methyl-3a,6a-dihydro-1H-pyrrolo[1,2,3]triazole-4,6-dione group.

8. The inhibitor of protein modification products formation according to clam 2 or 3, wherein R1 is a substituted or unsubstituted phenyl group and R2 is a hydroxymethyl group.

9. The inhibitor of protein modification products formation according to any one of claims 1 to 8, wherein the protein modification products is selected from AGEs, ALEs and combinations thereof.

10. The inhibitor of protein modification products formation according to claim 9, wherein the protein modification products is AGE.

11. The inhibitor of protein modification products formation according to claim 10, wherein AGE is pentosidine.

12. A renal tissue protecting agent comprising the inhibitor of protein modification products formation according to any one of claims 1 to 8.

13. A peritoneal dialysate comprising the inhibitor of protein modification products formation according to any one of claims 1 to 8.

14. A hemodialysis fluid comprising the inhibitor of protein modification products formation according to any one of claims 1 to 8.

15. A method for reducing the amount of carbonyl compounds in a liquid sample, which comprises contacting said liquid sample with the inhibitor of protein modification products formation according to any one of claims 1 to 8.

16. A method for suppressing the formation of protein modification products in the blood or peritoneal dialysate of a patient, which comprises contacting said blood or peritoneal dialysate with the inhibitor of protein modification products formation according to any one of claims 1 to 8.

17. A method for treating a disease mediated by the formation of protein modification products, which comprises administrating a therapeutically effective amount of the inhibitor of protein modification products formation according to any one of claims 1 to 8 to a patient in need of such treatment.

18. Use of the inhibitor of protein modification products formation according to any one of claims 1 to 8 for preparation of a medicament for treating a disease mediated by the formation of protein modification products.
